# EUROPEAN PATENT SPECIFICATION

(11) **EP 3 533 443 B1**
(45) Date of publication and mention of the grant of the patent: **10.07.2024**
(21) Application number: 17864720.2
(22) Date of filing: 27.10.2017
(51) Int. Cl.: A61K 9/70, A61K 31/4045, A61K 47/10, A61K 47/12, A61K 47/14, A61K 47/22, A61K 47/26, A61K 47/32, A61K 47/38

(54) **PERCUTANEOUS ABSORPTION FORMULATION FOR TREATING SLEEP DISORDERS**
FORMULIERUNG ZUR PERKUTANEN ABSORPTION ZUR BEHANDLUNG VON SCHLAFSTÖRUNGEN
FORMULATION POUR ABSORPTION PERCUTANÉE POUR LE TRAITEMENT DE TROUBLES DU SOMMEIL

(30) Priority: 31.10.2016 KR 20160143368; 25.10.2017 KR 20170138990
(43) Date of publication of application: 04.09.2019
(73) Proprietor: Sinsin Pharm Co., Ltd., Ansan-si, Gyeonggi-do 15434 (KR)
(72) Inventor: LEE, Jung Sik, Suwon-si Gyeonggi-do 16544 (KR); LEE, Sun Ho, Suwon-si Gyeonggi-do 16231 (KR); HAN, Mun Seok, Suwon-si Gyeonggi-do 16325 (KR); LEE, Woo Young, Suwon-si Gyeonggi-do 16279 (KR); KIM, Sang Lin, Seoul 01901 (KR); KIM, Han Ki, Yongin-si Gyeonggi-do 16822 (KR)
(74) Representative: Walcher, Armin
(86) International application number: PCT/KR2017/011961
(87) International publication number: WO 2018/080214

(56) References cited:
- WO-A1-96/30013
- WO-A2-2004/096118
- CN-A- 104 825 426
- CN-A- 104 940 172
- CN-A- 105 232 497
- DE-A1- 19 713 141
- JP-A- 2001 058 960
- JP-A- H10 182 455
- KR-A- 19947 002 724
- KR-A- 20110 118 525
- US-A- 5 385 736
- US-A1- 2005 042 271
- N. KANIKKANNAN ET AL: "Formulation and In Vitro Evaluation of Transdermal Patches of Melatonin", DRUG DEVELOPMENT AND INDUSTRIAL PHARMACY, vol. 30, no. 2, 3 November 2004 (2004-11-03), US, pages 205 - 212, XP055686528, ISSN: 0363-9045, DOI: 10.1081/DDC-120028716
- BENES L ET AL: "Transmucosal, oral controlled-release, and transdermal drug administration in human subjects: a crossover study with melatonin", JOURNAL OF PHARMACEUTICAL SCIENCES, AMERICAN PHARMACEUTICAL ASSOCIATION, US, vol. 86, no. 10, 1 October 1997 (1997-10-01), pages 1115 - 1119, XP002174139, ISSN: 0022-3549, DOI: 10.1021/JS970011Z
- H S GWAK ET AL: "Formulation and Evaluation of Melatonin Plasters", J. KOR. PHARM. SCI., vol. 32, no. 2, 2002, pages 107 - 112, XP055686581

## Description

### [Technical Field]

The present invention relates to a formulation of percutaneous absorption of melatonin, which contains melatonin as an active ingredient, to improve patient's medication adherence, enabling the drug to be stably maintained without forming a crystal, albeit its high concentration in an adhesive matrix, and can be used in a method of delivering the active ingredient into the body through the skin.

### [Background Art]

JPH10-182455 is directed to a melatonin-containing plaster.

Melatonin is a hormone secreted by the mammalian pineal gland. The circadian rhythm of melatonin secretion is photoperiodically regulated. The concentration of melatonin in plasma begins to increase at around 9:00 pm, reaches its peak from 2:00 am to 4:00 am, and returns back to its original level from 7:00 am to 9:00 am.

Melatonin has a sleep-inducing effect, and thus is used for treatment of insomniac patients and sleep disorder patients. However, melatonin, when administered orally, undergoes serious liver first-pass, has low bioavailability due to its short half-life (about 1 hour), and cause huge changes in blood concentration. Thus, it has been reported that the bioavailability of melatonin is only 20%.

Therefore, for effective treatment with melatonin, there is a need to develop a novel formulation which can avoid liver first-pass, can maintain the blood melatonin concentration at a constant level over a long period of time, and can be absorbed rapidly.

### [Disclosure]

### [Technical Problem]

A reservoir-type percutaneous absorption formulation comprises a gel-type drug-containing reservoir layer, a release layer for controlling drug release, and an adhesive material for attaching to the skin. However, there are problems such as, that the reservoir may be damaged during storage or use, and that if the reservoir is damaged during use by a patient, the drug may be absorbed at a level higher than its predetermined level, resulting in patient risk.

In addition, when an acrylic polymer is synthesized directly during the preparation of a monolithic-type formulation, a problem arises in that the toxicity and stability of an adhesive as an adhesive agent are not verified. This is risky and inefficient because percutaneous absorption formulation manufacturers generally produce percutaneous absorption formulations using commercialized adhesives.

Therefore, there is a need to develop a formulation for percutaneous absorption of melatonin, which can overcome the above-described disadvantages of a percutaneous absorption formulation, capable of controlling drug release, has high stability, convenient to store and use, and easy to manufacture.

This present invention is intended to provide a monolithic thin film-type formulation for percutaneous absorption of melatonin, which comprises a combination of raw materials with verified safety. Thus, it has high safety and is convenient to store and use. Unlike the conventional reservoir-type formulation, the monolithic percutaneous absorption formulation can store, stabilize, and control percutaneous absorption of drug.

### [Technical Solution]

The object underlying the invention is solved by providing a percutaneous absorption formulation according to claim 1. Preferred embodiments are specified in claims 2 to 8.

To solve the above problem, without taking a reservoir type, the present inventors have combined a suitable adhesive material with raw materials thereby completing a monolithic thin film-type formulation for percutaneous absorption of melatonin, which has improved drug stability and possible for drug to be delivered at a constant level through the skin.

### [Advantageous Effects]

The formulation for percutaneous absorption of melatonin according to the present invention enables controlled drug release, has high stability, is convenient to store and use, and is easy to manufacture. In addition, the percutaneous absorption formulation, according to the present invention, can significantly improve patient's medication compliance compared to an oral formulation, and at the same time, can deliver an effective amount of necessary drug to a patient, indicating that it can be advantageously used for the treatment of insomnia and sleep disorders. In addition, the amount of the formulation remaining in the skin after removal can be minimized.

### [Description of Drawings]

FIG. 1 is a sectional view of a monolithic percutaneous absorption formulation manufactured by the present invention. A is a supporting layer, B is a drug-containing adhesive layer, and C is a releasing layer.
FIG. 2 is a graph showing cumulative percutaneous absorption for Examples 18 to 23 (Examples 18-20, 22 and 23 are according to the invention; Example 21 is a reference example) and Comparative Examples 3 and 4.

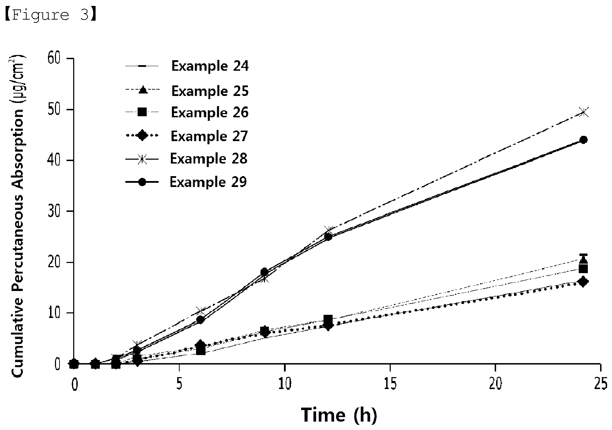

FIG. 3 is a graph showing cumulative percutaneous absorption for Examples 24 to 29 (Examples 26, 27 and 29 are according to the invention; Examples 24, 25 and 28 are reference examples).

### [Best Mode]

The present invention relates to a percutaneous absorption formulation comprising melatonin as an active ingredient, specifically to a monolithic formulation for percutaneous absorption of melatonin.

The percutaneous absorption formulation of the present invention comprises: (a) a drug-containing adhesive layer as claimed, which contains melatonin or a pharmaceutically acceptable salt as an active ingredient, and a polymeric adhesive agent; (b) a supporting layer; and (c) a releasing layer. It may be used for the treatment of insomnia and sleep disorders.

In the present invention, the drug-containing adhesive layer as claimed contains, in addition to melatonin or a pharmaceutically acceptable salt thereof, a polymeric adhesive agent, a solubilizing agent, a crystallization inhibitor, a percutaneous absorption enhancer, and an antioxidant.

As the polymeric adhesive agent that is used in the present invention, any pressure-sensitive adhesive may be used without limitation, but an acrylic adhesive is more preferable.

An acrylic adhesive that may be used in the present invention is an acrylic polymer adhesive agent composed of either acrylate or a copolymer of acrylate and vinyl acetate. The acrylic polymer adhesive agent may be one or two or more selected from the group consisting of (i) one having no functional group, (ii) one having a hydroxyl (-OH) group as a functional group, (iii) one having a carboxyl (-COOH) group as a functional group, and (iv) one having both a hydroxyl group and a carboxyl group as functional groups. Preferably, an acrylic adhesive having a carboxyl (-COOH) group or containing both a carboxyl group and a hydroxyl (-OH) group may be used.

Since melatonin has a moiety having non-covalent electrons in its structure, an adhesive having a hydroxyl group rich in electrons is advantageous for percutaneous absorption, but may have a problem in that it easily forms crystals. A non-functional acrylic adhesive may have a problem in that the adhesive easily forms crystals, because the solubility of the adhesive itself is lowered due to the functional group of melatonin.

On the other hand, an adhesive having a carboxylic group can solubilize the drug by interaction with the functional group of melatonin, and crystallization of the active ingredient component can be effectively inhibited by the use of little amount of a solubilizing agent and a crystallization inhibitor.

In addition, the polymeric adhesive agent that is used in the present invention may be an adhesive agent comprising a hydrophobic polymer. As a hydrophobic polymer, one or two or more can be selected from the group consisting of polyisoprene, polyisobutylene, polybutadiene, a polystyrene-butadiene copolymer, a polystyrene-isoprene copolymer, a styrene-isoprene-styrene block copolymer, a styrene-butadiene-styrene block copolymer, butyl rubber, natural rubber, an ethylene-vinyl acetate copolymer, polysiloxane, and methacrylic acid-based polymers.

The polymeric adhesive agent may further comprise a tackifying resin and a plasticizer. In this case, the hydrophobic polymer may be contained in an amount of 20 to 60 wt%, the tackifying resin may be contained in an amount of 20 to 50 wt%, and the plasticizer may be contained in an amount of 2 to 30 wt%.

An acrylic adhesive is preferable in terms of solubility of drug or adhesion.

In the present invention, a solubilizing agent is used for stable solubilization of melatonin in percutaneous absorption formulations. The solubilizing agent is one or two or more selected from the group consisting of N-methylpyrrolidone, dipropylene glycol, propylene glycol, propylene carbonate, ethoxydiglycol, diethylene glycol monoethyl ether, triacetin, triethyl citrate, triethanolamine, tromethamine, bis-Tris, aminomethyl propanediol, aminoethyl propanediol, and polyoxyethylene sorbitan monooleate.

Melatonin is highly soluble in substances having both hydrophilic and hydrophobic properties, due to the molecular characteristics of melatonin having both hydrophilic and hydrophobic moieties. The solubilizing agent of the present invention is used in an amount of 1 to 30 wt%, preferably 1 to 15 wt%, based on the total weight of the drug-containing adhesive layer. If the solubilizing agent is used in an amount larger than 30 wt%, it may damage the user's skin, and may also reduce the physical strength of the percutaneous absorption formulation.

In the present invention, a crystallization inhibitor of the drug is contained in order to prevent the crystallization of melatonin in the percutaneous absorption formulation. The crystallization inhibitor is an amino acrylic methacrylate copolymer and/or polyvinylpyrrolidone.

The crystallization inhibitor is used in an amount of 0.05 to 5 wt%, preferably 0.05 to 2.5 wt%, based on the total weight of the drug-containing adhesive layer. The crystallization inhibitor is a polymer, and hence if it is used in an amount larger than 5 wt%, problems such as sticking with an acrylic adhesive may occur, and adhesive strength may also be reduced.

In the present invention, the percutaneous absorption formulation further comprises a percutaneous absorption enhancer. As the percutaneous absorption enhancer, a C₈₋₁₈ aliphatic derivative is used, that is one or two or more selected from the group consisting of glycerol lauryl alcohol, oleyl alcohol, isopropyl myristate, sorbitan monooleate, propylene glycol monolaurate, propylene glycol monooleate, oleoyl macrogolglycerides, oleic acid, lauroyl macrogol glyceride, linoleoyl macrogol glyceride, propylene glycol dicaprylate/caprate, sorbitan monostearate, glycerol monooleate, glycerol monolaurate, sorbitan monolaurate, lauryl lactate, PEG-8 caprylic/capric triglycerides, polyoxyethylene sorbitan monolaurate, corn oil PEG-8 esters, and corn oil PEG-6 esters.

More preferably, as the C₈₋₁₈ aliphatic derivative, one or two or more can be selected from the group consisting of glycerol monooleate, glycerol monolaurate, propylene glycol monolaurate, sorbitan monooleate, sorbitan monolaurate, corn oil PEG-8 esters, and corn oil PEG-6 esters.

The percutaneous absorption enhancer of the present invention is used in an amount of 1 to 30 wt%, preferably 1 to 15 wt%, based on the total weight of the drug-containing adhesive layer. If the percutaneous absorption enhancer is used in an amount larger than 30 wt%, then it will no longer improve the percutaneous absorption of the drug, resulting in a decrease in the physical strength of the percutaneous absorption formulation, and in some cases, can cause skin troubles in the user.

The percutaneous absorption formulation of the present invention further comprises an antioxidant in order to inhibit degradation and denaturation in the percutaneous absorption formulation. Since melatonin also acts as an oxygen radical scavenger *in vivo,* the percutaneous absorption formulation contains an antioxidant to suppress oxidation.

As the antioxidant, one or two or more are selected from the group consisting of butyl hydroxy toluene, butyl hydroxy anisole, propyl galate, ascorbic acid, tocopherol, tocopherol acetate, and ascorbyl palmitate. Preferably, butyl hydroxy toluene, tocopherol, tocopherol acetate, or butyl hydroxy anisole.

The antioxidant is used in an amount of 0.1 to 5 wt%, preferably 0.1 to 1 wt%, based on the total weight of the drug-containing adhesive layer. If the antioxidant is used in an amount larger than 5 wt%, the antioxidant effect is no longer improved.

In the present invention, the drug-containing adhesive layer contains melatonin or a pharmaceutically acceptable salt thereof in an amount of 3 to 20 wt% based on the total weight of the drug-containing adhesive layer. Meanwhile, the drug-containing adhesive layer further contains, in addition to melatonin or a pharmaceutically acceptable salt thereof, a solubilizing agent, a crystallization inhibitor, a percutaneous absorption enhancer and an antioxidant in the above-described amounts, and further contains a suitable amount (50 to 94.85 wt%) of a polymeric adhesive agent.

In the present invention, the supporting layer is used to prevent melatonin from being lost during attachment to the skin or storage, and is made of a thin, flexible material, that causes no skin allergic reaction. The supporting layer may be made of a material, such as polyethylene terephthalate (PET), polyethylene (PE), polypropylene (PP), ethylene-vinyl acetate (EVA) copolymer, nylon, or the like. A film made of this material may be used alone, or in a laminated film form obtained by laminating two or more films. In addition, when two or more films are laminated, a film having aluminum deposited may be used for light shielding and prevention of moisture penetration. Furthermore, in order to facilitate patch formation, nonwoven fabric, cotton cloth, fabric or the like may be laminated on to this film, or the above-mentioned fabric may also be used alone.

In the present invention, the releasing layer is not particularly limited, as long as it protects the drug-containing product during packaging or storage of the product and provides convenience so that it can be easily removed when using the product. It may be either a film produced from polyester, polyvinyl chloride, polyvinylidene chloride, polyethylene terephthalate, polyethylene, ethylene vinyl acetate or the like, or a film obtained by laminating polyolefin on paper such as wood-free paper or glassine paper. The adhesive layer-contacting surface of the releasing film is coated with silicone resin or fluorine resin. As the release layer, a polyethylene terephthalate film showing excellent long-term stability of the drug is preferably used.

### [Mode for Invention]

### [Examples]

Hereinafter, the present invention will be described in detail with reference to examples. However, the following examples are only illustrative of the present invention, and the scope of the present invention is not limited to the following examples.

### 1. Examples 1 to 5 (reference examples) and Comparative Examples 1 to 2

10 wt% melatonin was placed in a 70 mL vial and dissolved in methanol, and then 90 wt% adhesive was added thereto, followed by mixing in a roll mixer for 2 hours. Next, the mixture was allowed to stand for 30 minutes to remove bubbles, after which a silicone-treated polyethylene terephthalate release film was placed on a coating machine, and the mixed adhesive was dried, and then coated onto the release film to 70 µm. Thereafter, the resultant was dried in an oven at 80°C for 3 minutes, and then a supporting layer film was laminated thereon and compressed using a roller press. The resultant was cut by means of a patch cutter, thereby manufacturing a patch having an area of 10 cm². The contents of the components according to each Example are shown in Table 1 below. Next, the physical properties of the patch and crystal formation were observed, and a percutaneous absorption test was performed. The observation and test results are also shown in Table 1 below.

### Percutaneous absorption test

Phosphate buffered saline solution (PBS solution, pH=7.4) as a receptor was placed in a Franz-type diffusion cell and stirred with a magnetic stirrer at a constant stirring speed of 600 rpm while maintaining 32°C which is similar to skin temperature, thereby removing dissolved gas from the solution. Next, the patch cut in accordance with the upper donor cell of the Franz-type diffusion cell was attached to a membrane for percutaneous absorption (Strat-M, Merck KGaA, Darmstadt, Germany) and mounted in the Franz-type diffusion cell. Next, the solution in the receptor compartment was sampled at predetermined time intervals, and a fresh buffer solution was supplemented in an amount equal to the sampled amount. The sample was analyzed by high-performance liquid chromatography (HPLC) under the following conditions:

### Analysis conditions

Column: C18, 4.6 mm, 5 µm (Agilent eclips SB-18);
Mobile phase: Methanol: water = 35:65 (v/v);
Flow rate: 1 mL/min;
Column temperature: 35°C;
Amount injected: 20 ul;
Detection: UV 229 nm.

**[Table 1]**

| Classification | | Examples (wt%) | | | | | Comparative Examples (wt%) | |
|---|---|---|---|---|---|---|---|---|
| | | 1 | 2 | 3 | 4 | 5 | 1 | 2 |
| Melatonin | | 10 | 10 | 10 | 10 | 10 | 10 | 10 |
| Adhesive | Duro-Tak^{™} 87-9301 | 90 | | | | | | |
| | Duro-Tak^{™} 87-2516 | | 90 | | | | | |
| | Duro-Tak^{™} 87-2074 | | | 90 | | | | |
| | Duro-Tak^{™} 87-4098 | | | | 90 | | | |
| | Duro-Tak^{™} 87-2852 | | | | | 90 | | |
| | Duro-Tak^{™} 87-608A (PIB) | | | | | | 90 | |
| | Sanicare HM 8663 (SIS) | | | | | | | 90 |
| Patch appearance | Initial | Colorless translucent | Yellow translucent | Colorless transparent | White translucent | Colorless transparent | Colorless transparent | Colorless transparent |
| | After 1 month | Yellow translucent | Yellow translucent | Yellow transparent | Yellow translucent | Yellow transparent | Yellow transparent | Yellow transparent |
| Physical properties and adhesive strength | | Good | Good | Good | Good | Good | Good | Good |
| Percutaneous absorption (µg/cm²/24h) | | 0.37 | 1.01 | 0.44 | 0.4 | 0.23 | 1.12 | 0.89 |
| Crystal formation (1 month) | | Crystal precipitation | Crystal precipitation | None | Crystal precipitation | None | Crystal precipitation | Crystal precipitatio n |

| Adhesive agent used | | | | | | | | |
|---|---|---|---|---|---|---|---|---|
| Name of raw material | | Kind | | | | Functional group | | |
| Duro-Tak^{™} 87-9301 | | Acrylic polymer adhesive agent | | | | Non | | |
| Duro-Tak^{™} 87-2516 | | Acrylic polymer adhesive agent | | | | -OH | | |
| Duro-Tak^{™} 87-2074 | | Acrylic polymer adhesive agent | | | | -COOH/-OH | | |
| Duro-Tak^{™} 874098 | | Acryl-vinyl acetate-based polymeric adhesive agent | | | | Non | | |
| Duro-Tak^{™} 87-2852 | | Acrylic polymer adhesive agent | | | | -COOH | | |
| Duro-Tak^{™} 87-608A | | Polyisobutylene adhesive agent | | | | N/A | | |
| Sanicare HM 8663 | | Styrene-isoprene-styrene copolymer adhesive agent | | | | N/A | | |

Duro-Tak^{™} 87-2516 was the adhesive showing the highest percutaneous absorption, but formed a crystalline precipitate. The adhesives which formed no crystalline precipitate were Duro-Tak^{™} 87-2074 and Duro-Tak^{™} 87-2852, and Duro-Tak^{™} 87-2074 showed higher percutaneous absorption than Duro-Tak^{™} 87-2852. The rubber-based adhesives of the Comparative Examples showed a good percutaneous absorption, but formed a crystalline precipitate.

### Examples 6 to 10 (reference examples)

Melatonin and a solubilizing agent were placed in a 70 mL vial and dissolved in methanol, and then an adhesive was added thereto, followed by mixing in a roll mixer for 2 hours. Next, the mixture was allowed to stand for 30 minutes to remove bubbles, after which a silicone-treated polyethylene terephthalate release film was placed on a coating machine, and the mixed adhesive was dried, and then coated on the release film to 70 um. Thereafter, the resultant was dried in an oven at 80°C for 3 minutes, and then a support layer film was laminated thereon and compressed using a roller press. The resultant was cut by using a patch cutter, thereby manufacturing a patch having an area of 10 cm². The contents of the components according to each Example are shown in Table 2 below. Next, the physical properties of the patch and crystal formation were observed. The observation and test results are also shown in Table 2 below.

**[Table 2]**

| Classification | | Examples (wt%) | | | | |
|---|---|---|---|---|---|---|
| | | 6 | 7 | 8 | 9 | 10 |
| Melatonin | | 10 | 10 | 10 | 10 | 10 |
| Adhesive | Duro-Tak^{™} 87-2074 | 85 | 85 | 85 | 85 | 85 |
| Solubilizing agent | NMP | 5 | | | | |
| | Transcutol CG | | 5 | | | |
| | Transcutol P | | | 5 | | |
| | Labrasol | | | | 5 | |
| | DPG | | | | | 5 |
| Patch appearance | Initial | Colorless translucent | Yellow translucent | Colorless transparent | White translucent | Colorless transparent |
| | After 1 month | Yellow translucent | Yellow translucent | Yellow transparent | Yellow translucent | Yellow transparent |
| Physical properties and adhesive strength | | Good | Good | Good | Good | Good |
| Crystal formation (after 1 month) (microscopic observation) | | Fine crystal formation | Fine crystal formation | Crystal formation | Crystal formation | Fine crystal formation |
| NMP: N-methyl pyrrolidone; | | | | | | |
| Transcutol CG: ethoxydiglycol; | | | | | | |
| Transcutol P: diethylene glycol monoethyl ether, | | | | | | |
| Labrasol: PEG-8 caprylic/capric glyceride; | | | | | | |
| DPG: dipropylene glycol | | | | | | |

When the manufactured patches were observed, N-methyl pyrrolidone, Transcutol CG and dipropylene glycol showed no crystal formation in visual observation, but showed fine crystal formation in microscopic observation, and yellowing in color in the manufactured patches has appeared. Thus, in order to effectively inhibit crystal formation in a patch, an additional study on a crystallization inhibitor was conducted, and an antioxidant formulation study was conducted to prevent yellowing.

### Examples 11 to 17 (reference examples)

A crystallization inhibitor was placed and dissolved in a 70 mL vial, and then a solubilizing agent, an antioxidant and melatonin were added and dissolved, after which an adhesive was added thereto, followed by mixing in a roll mixer for 2 hours. Next, the mixture was allowed to stand for 30 minutes to remove bubbles, after which a silicone-treated polyethylene terephthalate release film was placed on a coating machine, and the mixed adhesive was dried, and then coated on the release film to 70 um. Thereafter, the resultant was dried in an oven at 80°C for 3 minutes, and then a support layer film was laminated thereon and compressed using a roller press. The resultant was cut by using a patch cutter, thereby manufacturing a patch having an area of 10 cm². The contents of the components according to each Example are shown in Table 3 below. Next, the physical properties of the patch and crystal formation were observed. The observation and test results are also shown in Table 3 below.

**[Table 3]**

| Classification | | Examples (wt%) | | | | | | |
|---|---|---|---|---|---|---|---|---|
| | | 11 | 12 | 13 | 14 | 15 | 16 | 17 |
| Melatonin | | 10 | 10 | 10 | 10 | 10 | 10 | 10 |
| Adhesive | Duro-Tak^{™} 87-2074 | 83 | 83 | 83 | 83 | 83 | 83 | 83 |
| Solubilizing agent | DPG | 5 | 5 | 5 | 5 | 5 | | 5 |
| Antioxidant | BHT | 0.5 | | 0.5 | 0.5 | 0.5 | 0.5 | 0.5 |
| | TA | | 05 | | | | | |
| Crystallization inhibitor | PVP | 1.5 | 1.5 | | | | | |
| | EudragitL-100 | | | 1.5 | | | | |
| | Eudragit S-100 | | | | 1.5 | | | |
| | Plastoid B | | | | | 1.5 | | |
| | Eudragit E-100 | | | | | | 1.5 | |
| | Klucel-LF | | | | | | | 1.5 |
| Patch appearance | Initial | Colorless transparent | Colorless transparent | Colorless transparent | Colorless transparent | Colorless transparent | Colorless transparent | Colorless transparent |
| | After 1 month | Colorless transparent | Colorless transparent | Colorless transparent | Colorless transparent | Colorless transparent | Colorless transparent | Colorless transparent |
| Physical properties and adhesive strength | | Good | Good | Good | Good | Good | Good | Good |
| Crystal formation (after 1 month) (microscopic observation) | | None | None | Fine crystal formation | Fine crystal formation | Fine crystal formation | None | Crystal formation |
| DPG: dipropylene glycol; | | | | | | | | |
| BHT: butyl hydroxyl toluene; | | | | | | | | |
| TA: tocopherol acetate; | | | | | | | | |
| PVP: PolyvinylPyrrolidone; | | | | | | | | |
| Eudragit RL-100: Ammonio Methacrylate Copolymer, Type A | | | | | | | | |
| Eudragit RS-100: Ammonio Methacrylate Copolymer, Type B | | | | | | | | |
| Plastoid B: Butyl methacrylic methacrylate copolymer, | | | | | | | | |
| Eudragit E-100: Amino Methacrylate Copolymer; | | | | | | | | |
| Klucel-LF: Hydroxypropyl cellulose | | | | | | | | |

In the test results, the antioxidant inhibited the oxidation of the active ingredient component, regardless of the class of the antioxidant component, and thus yellowing in the drug layer of each patch did not appear. In addition, polyvinyl pyrrolidone (PVP) and the amino methacrylate copolymer (Eudragit E-100) showed the highest inhibition of crystallization of the active ingredient component.

### Examples 18 to 23 (Examples 18-20, 22 and 23 are according to the invention; Example 21 is a reference example) and Comparative Example 3

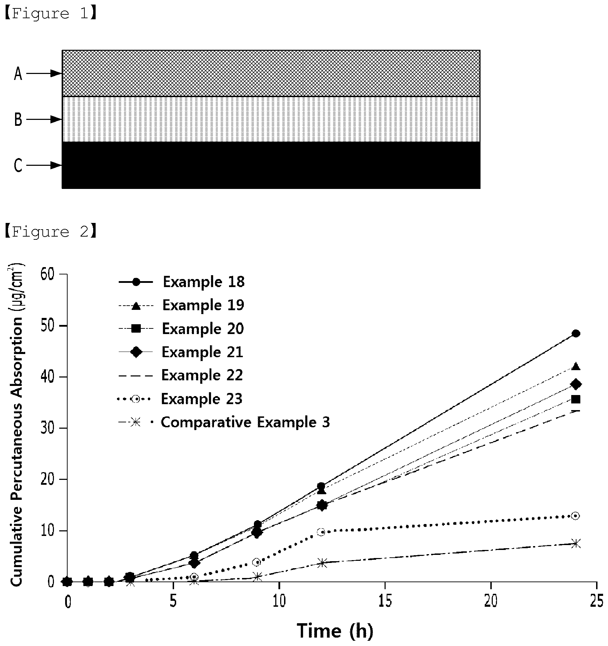

A crystallization inhibitor was placed and dissolved in a 70 mL vial, and then a solubilizing agent, an antioxidant, a percutaneous absorption enhancer and melatonin were added thereto and dissolved, after which an adhesive was added thereto, followed by mixing in a roll mixer for 2 hours. Next, the mixture was allowed to stand for 30 minutes to remove bubbles, after which a silicone-treated polyethylene terephthalate release film was placed on a coating machine, and the mixed adhesive was dried, and then coated on the release film to 70 um. Thereafter, the resultant was dried in an oven at 80°C for 3 minutes, and then a support layer film was laminated thereon and compressed using a roller press. The resultant was cut by means of a patch cutter, thereby manufacturing a patch having an area of 10 cm². The contents of the components according to each Example are shown in Table 4 below. Next, the physical properties of the patch and crystal formation were observed, and a percutaneous absorption test was performed. The observation and test results are also shown in Table 4 below and FIG. 2.

**[Table 4]**

| Classification | | Examples (wt%) | | | | | | Comparative Example (wt%) |
|---|---|---|---|---|---|---|---|---|
| | | 18 | 19 | 20 | 21 | 22 | 23 | 3 |
| Melatonin | | 10 | 10 | 10 | 10 | 10 | 10 | 10 |
| Adhesive | Duro-Tak^{™} 87-2074 | 78 | 78 | 78 | 78 | 80.5 | 83 | 83 |
| Solubilizing agent | DPG | 5 | 5 | 5 | 5 | 5 | 5 | 5 |
| Antioxidant | TA | 0.5 | 0.5 | 0.5 | 0.5 | 0.5 | 0.5 | 0.5 |
| Crystallization inhibitor | PVP | 1.5 | 1.5 | 1.5 | 1.5 | 1.5 | 1.5 | 1.5 |
| Percutaneous absorption enhancer | GML | 5 | | | | 2.5 | 5 | |
| | GMO | | 5 | | | | | |
| | SMO | | | 5 | | | | |
| | GMC | | | | 5 | | | |
| Percutaneous absorption (µg/cm²/24h) | | 47.82 | 42.15 | 34.23 | 37.95 | 33.27 | 13 | 7.4 |
| Patch appearance | Initial | Colorless transparent | Colorless transparent | Colorless transparent | Colorless transparent | Colorless transparent | Colorless transparent | Colorless transparent |
| | After 1 month | Colorless transparent | Colorless transparent | Colorless transparent | Colorless transparent | Colorless transparent | Colorless transparent | Colorless transparent |
| Physical properties and adhesive strength | | Good | Good | Good | Good | Good | Good | Good |
| Crystal formation (after 1 month) (microscopic observation) | | None | None | None | None | None | None | None |
| DPG: dipropylene glycol; | | | | | | | | |
| TA: tocopherol acetate; | | | | | | | | |
| PVP: polyvinyl pyrrolidone; | | | | | | | | |
| GML: glycerol monolaurate; | | | | | | | | |
| GMO: glycerol monooleate; | | | | | | | | |
| SMO: sorbitan monooleate; | | | | | | | | |
| GMC: glycerol monocaprylate | | | | | | | | |

As the test results shown in Table 4 above, the manufactured patches effectively achieved the percutaneous absorption of melatonin.

It shows that Examples 18 to 22 showed an increase in percutaneous absorption due to the use of the percutaneous absorption enhancer, compared to Comparative Example 3. In addition, it shows that Examples 18 to 22 showed changes in percutaneous absorption depending on the kind of additive (such as percutaneous absorption enhancer) and the amount of additive used.

### Examples 24 to 29 (Examples 26, 27 and 29 are according to the invention; Examples 24, 25 and 28 are reference examples)

A crystallization inhibitor was placed and dissolved in a 70 mL vial, and then a solubilizing agent, an antioxidant, a percutaneous absorption enhancer and melatonin were added thereto and dissolved, after which an adhesive was added thereto, followed by mixing in a roll mixer for 2 hours. Next, the mixture was allowed to stand for 30 minutes to remove bubbles, after which a silicone-treated polyethylene terephthalate release film was placed on a coating machine, and the mixed adhesive was dried, and then coated on the release film to 70 um. Thereafter, the resultant was dried in an oven at 80°C for 3 minutes, and then a support layer film was laminated thereon and compressed using a roller press. The resultant was cut by means of a patch cutter, thereby manufacturing a patch having an area of 10 cm². The contents of the components according to each Example are shown in Table 5 below. Next, the physical properties of the patch and crystal formation were observed, and a percutaneous absorption test was performed. The observation and test results are also shown in Table 5 below.

**[Table 5]**

| Classification | | Examples (wt%) | | | | | |
|---|---|---|---|---|---|---|---|
| | | 24 | 25 | 26 | 27 | 28 | 29 |
| Melatonin | | 10 | 10 | 10 | 10 | 10 | 10 |
| Adhesive | Duro-Tak^{™} 87-2074 | 78 | 78 | 78 | 78 | 73 | 73 |
| Solubilizing agent | DPG | 5 | 5 | 5 | 5 | 5 | 5 |
| Antioxidant | TA | 0.5 | 0.5 | 0.5 | 0.5 | 0.5 | 0.5 |
| Crystallization inhibitor | PVP | 1.5 | 1.5 | 1.5 | 1.5 | 1.5 | 1.5 |
| Percutaneous absorption enhancer | LNA | 5 | | | | | |
| | MYA | | 5 | | | 10 | |
| | OLE | | | 5 | | | 10 |
| | PGML | | | | 5 | | |
| Percutaneous absorption (µg/cm²/24h) | | 33.27 | 41.04 | 37.25 | 32.13 | 98.52 | 87.41 |
| Patch appearance | Initial | Colorless transparent | Colorless transparent | Colorless transparent | Colorless transparent | Colorless transparent | Colorless transparent |
| | After 1 month | Colorless transparent | Colorless transparent | Colorless transparent | Colorless transparent | Colorless transparent | Colorless transparent |
| Physical properties and adhesive strength | | Good | Good | Good | Good | Good | Good |
| Crystal formation (after 1 month) (microscopic observation) | | None | None | None | None | None | None |
| LNA: linoleic acid; | | | | | | | |
| MYA: myristic acid; | | | | | | | |
| OLA: oleic acid; | | | | | | | |
| PGML: propylene glycol monolaurate | | | | | | | |

As shown in Table 5 above, the manufactured patches easily achieved percutaneous absorption when the C₈₋₁₈ aliphatic derivative was used as the percutaneous absorption enhancer, like the case of Examples 23 to 28.

### [Industrial Applicability]

The formulation for percutaneous absorption of melatonin according to the present invention enables controlled drug release, has high stability, convenient to store and use, and is easy to manufacture. In addition, the percutaneous absorption formulation according to the present invention can significantly improve patient's medication compliance compared to an oral formulation, and at the same time, it can be used in a method of delivering an effective amount of a necessary drug to a patient, indicating that it can be advantageously used in a method of treatment of insomnia and sleep disorders.

## Claims

1. A percutaneous absorption formulation comprising: (a) a drug-containing adhesive layer which contains melatonin or a pharmaceutically acceptable salt thereof as an active ingredient, a solubilizing agent, a crystallization inhibitor, a percutaneous absorption enhancer, an antioxidant, and a polymeric adhesive agent; (b) a support layer; and (c) a release layer,
wherein the drug-containing adhesive layer contains, based on the total weight of the drug-containing adhesive layer, 3.0 to 20 wt% of melatonin or the pharmaceutically acceptable salt thereof, 1 to 30 wt% of the solubilizing agent, 0.05 to 5 wt% of the crystallization inhibitor, 1 to 30 wt% of the percutaneous absorption enhancer, 0.1 to 5 wt% of the antioxidant, and 50 to 94.85 wt% of the polymeric adhesive agent,
wherein the solubilizing agent is one or two or more selected from the group consisting of N-methylpyrrolidone, dipropylene glycol, propylene glycol, propylene carbonate, ethoxydiglycol, diethylene glycol monoethyl ether, triacetin, triethyl citrate, triethanolamine, tromethamine, bis-Tris, aminomethyl propanediol, aminoethyl propanediol, and polyoxyethylene sorbitan monooleate,
wherein the crystallization inhibitor for inhibiting crystal formation of the drug is polyvinylpyrrolidone and/or an amino acrylic methacrylate copolymer,
wherein the percutaneous absorption enhancer is a C8-18 aliphatic derivative,
wherein the C8-18 aliphatic derivative is one or two or more selected from the group consisting of glycerol lauryl alcohol, oleyl alcohol, isopropyl myristate, sorbitan monooleate, propylene glycol monolaurate, propylene glycol monooleate, oleoyl macrogolglycerides, oleic acid, lauroyl macrogol glyceride, linoleoyl macrogol glyceride, propylene glycol dicaprylate/caprate, sorbitan monostearate, glycerol monooleate, glycerol monolaurate, sorbitan monolaurate, lauryl lactate, PEG-8 caprylic/capric triglycerides, polyoxyethylene sorbitan monolaurate, corn oil PEG-8 esters, and corn oil PEG-6 esters, and
wherein the antioxidant is one or two or more selected from the group consisting of butyl hydroxy toluene, butyl hydroxy anisole, propyl galate, ascorbic acid, tocopherol, tocopherol acetate, and ascorbyl palmitate.

2. The percutaneous absorption formulation of claim 1, wherein the polymeric adhesive agent is an acrylic adhesive.

3. The percutaneous absorption formulation of claim 1, wherein the polymeric adhesive agent is an acrylic polymer adhesive agent composed of either acrylate or a copolymer of acrylate and vinyl acetate, wherein the acrylic polymer adhesive agent is one or two or more selected from the group consisting of (i) one having no functional group, (ii) one having a hydroxyl (-OH) group as a functional group, (iii) one having a carboxyl (-COOH) group as a functional group, and (iv) one having both a hydroxyl group and a carboxyl group as functional groups.

4. The percutaneous absorption formulation of claim 1, wherein the polymeric adhesive agent is an acrylic polymer adhesive agent composed of either acrylate or a copolymer of acrylate and vinyl acetate, wherein the acrylic polymer adhesive agent is (i) one having a carboxyl (-COOH) group as a functional group, or (ii) one having both a hydroxyl group and a carboxyl group as functional groups.

5. The percutaneous absorption formulation of claim 1, wherein the polymeric adhesive agent is an adhesive agent which comprises a hydrophobic polymer, wherein the hydrophobic polymer is one or two or more selected from the group consisting of polyisoprene, polyisobutylene, polybutadiene, polystyrene-butadiene copolymer, polystyrene-isoprene copolymer, styrene-isoprene-styrene block copolymer, styrene-butadiene-styrene block copolymer, butyl rubber, natural rubber, ethylene-vinyl acetate copolymer, polysiloxane, and methacrylic acid-based polymers.

6. The percutaneous absorption formulation of claim 5, wherein the polymeric adhesive agent further comprises a tackifying resin and a plasticizer, and comprises 20 to 60 wt% of the hydrophobic polymer, 20 to 50 wt% of the tackifying resin, and 2 to 30 wt% of the plasticizer.

7. The percutaneous absorption formulation of claim 1, wherein the support layer is one selected from the group consisting of a polyethylene terephthalate (PET) film, a polyethylene (PE) film, a polypropylene (PP) film, an ethylene vinyl acetate (EVA) film, a nylon film, a nonwoven fabric/PET laminate, a PET/PE laminate, and a PET/EVA laminate.

8. The percutaneous absorption formulation of claim 1, wherein the release layer is a release layer obtained by surface-treating one selected from the group consisting of a polyester film, a polyvinyl chloride film, a polyvinylidene chloride film, a polyethylene terephthalate (PET) film, a polyethylene (PE) film, a polyethylene/paper laminate, a PET/PE laminate, and a PET/EVA laminate, the surface-treating one being treated with a silicone or fluorine treatment agent.

## Patentansprüche

1. Perkutane Absorptionsformulierung, umfassend: (a) eine arzneimittelhaltige Haftschicht, die Melatonin oder ein pharmazeutisch akzeptables Salz davon als Wirkstoff, ein Solubilisierungsmittel, einen Kristallisationsinhibitor, einen perkutanen Absorptionsverstärker, ein Antioxidantionsmittel und ein polymeres Haftmittel; (b) eine Trägerschicht; und (c) eine Trennschicht enthält,
wobei die arzneimittelhaltige Haftschicht, bezogen auf das Gesamtgewicht der arzneimittelhaltigen Haftschicht, 3,0 bis 20 Gew.-% Melatonin oder das pharmazeutisch akzeptable Salz davon, 1 bis 30 Gew.-% von dem Solubilisierungsmittel, 0,05 bis 5 Gew.-% von dem Kristallisationsinhibitor, 1 bis 30 Gew.-% von dem perkutanen Absorptionverstärker, 0,1 bis 5 Gew.-% des Antioxidationsmittels und 50 bis 94,85 Gew.-% von dem polymeren Haftmittel enthält,
wobei das Solubilisierungsmittel eines oder zwei oder mehr ausgewählt aus der Gruppe bestehend aus N-Methylpyrrolidon, Dipropylenglykol, Propylenglykol, Propylencarbonat, Ethoxydiglykol, Diethylenglykolmonoethylether, Triacetin, Triethylcitrat, Triethanolamin, Tromethamin, bis-Tris, Aminomethylpropandiol, Aminoethylpropandiol und Polyoxyethylen-Sorbitan-Monooleat ist,
wobei der Kristallisationsinhibitor zur Hemmung der Kristallbildung des Arzneimittels Polyvinylpyrrolidon und/oder ein Aminoacrylatmethacrylat-Copolymer ist,
wobei der perkutane Absorptionsverstärker ein aliphatisches C8-18-Derivat ist,
wobei das aliphatische C8-18-Derivat ein oder zwei oder mehr ausgewählt aus der Gruppe bestehend aus Glycerinlaurylalkohol, Oleylalkohol, Isopropylmyristat, Sorbitanmonooleat, Propylenglykolmonolaurat, Propylenglykol-Monooleat, Oleoylmakrogolglyceride, Ölsäure, Lauroylmakrogolglycerid, Linoleoylmakrogolglycerid, Propylenglykol-Dicaprylat/Caprat, Sorbitanmonostearat, Glycerolmonooleat, Glycerolmonolaurat, Sorbitanmonolaurat, Lauryllactat, PEG-8-Capryl/Caprin-Triglyceride, Polyoxyethylen-Sorbitanmonolaurat, Maisöl-PEG-8-Ester und Maisöl-PEG-6-Ester ist, und
wobei das Antioxidationsmittel eines oder zwei oder mehr ausgewählt aus der Gruppe bestehend aus Butylhydroxytoluol, Butylhydroxyanisol, Propylgalat, Ascorbinsäure, Tocopherol, Tocopherolacetat und Ascorbylpalmitat ist.

2. Perkutane Absorptionsformulierung nach Anspruch 1, wobei das polymere Haftmittel ein Acrylatkleber ist.

3. Perkutane Absorptionsformulierung nach Anspruch 1, wobei das polymere Haftmittel ein Acrylpolymer-Haftmittel, das entweder aus Acrylat oder aus einem Copolymer von Acrylat und Vinylacetat aufgebaut ist, wobei das Acrylpolymer-Haftmittel eines oder zwei oder mehr ausgewählt aus der Gruppe bestehend aus (i) einem ohne funktionelle Gruppe, (ii) einem mit einer Hydroxyl(-OH)-Gruppe als einer funktionellen Gruppe, (iii) einem mit einer Carboxyl(-COOH)-Gruppe als einer funktionellen Gruppe und (iv) einem mit sowohl einer Hydroxylgruppe als auch einer Carboxylgruppe als funktionelle Gruppen ist.

4. Perkutane Absorptionsformulierung nach Anspruch 1, wobei das polymere Haftmittel ein Acrylpolymer-Haftmittel, das entweder aus Acrylat oder aus einem Copolymer von Acrylat und Vinylacetat aufgebaut ist, wobei das Acrylpolymer-Haftmittel (i) eines mit einer Carboxyl(-COOH)-Gruppe als einer funktionellen Gruppe oder (ii) eines mit sowohl einer Hydroxylgruppe als auch einer Carboxylgruppe als funktionelle Gruppen ist.

5. Perkutane Absorptionsformulierung nach Anspruch 1, wobei das polymere Haftmittel ein Haftmittel ist, das ein hydrophobes Polymer umfasst, wobei das hydrophobe Polymer eines oder zwei oder mehr ausgewählt aus der Gruppe bestehend aus Polyisopren, Polyisobutylen, Polybutadien, Polystyrol-Butadien-Copolymer, Polystyrol-Isopren-Copolymer, Styrol-Isopren-Styrol-Block-Copolymer, Styrol-Butadien-Styrol-Block-Copolymer, Butylkautschuk, Naturkautschuk, Ethylen-Vinylacetat-Copolymer, Polysiloxan und Polymere auf Methacrylsäurebasis ist.

6. Perkutane Absorptionsformulierung nach Anspruch 5,
wobei das polymere Haftmittel weiter ein klebrigmachendes Harz und einen Weichmacher umfasst und 20 bis 60 Gew.-% des hydrophoben Polymers, 20 bis 50 Gew.-% des klebrigmachenden Harzes und 2 bis 30 Gew.-% des Weichmachers umfasst.

7. Perkutane Absorptionsformulierung nach Anspruch 1, wobei die Trägerschicht eine ausgewählt aus der Gruppe bestehend aus einer Polyethylenterephthalat-(PET)-Folie, einer Polyethylen-(PE)-Folie, einer Polypropylen-(PP)-Folie, einer Ethylenvinylacetat-(EVA)-Folie, einer Nylonfolie, einem Vliesstoff/PET-Laminat, einem PET/PE-Laminat und einem PET/EVA-Laminat ist.

8. Perkutane Absorptionsformulierung nach Anspruch 1, wobei die Trennschicht eine Trennschicht ist, die durch Oberflächenbehandlung mit einer ausgewählt aus der Gruppe bestehend aus einer Polyesterfolie, einer Polyvinylchloridfolie, einer Polyvinylidenchloridfolie, einer Polyethylenterephthalat-(PET)-Folie, einer Polyethylen-(PE)-Folie, einem Polyethylen/Papier-Laminat, einem PET/PE-Laminat und einem PET/EVA-Laminat erhalten wird, wobei die Oberflächenbehandlung eine ist, bei der mit einem Silikon- oder Fluorbehandlungsmittel behandelt wird.

## Revendications

1. Une formulation pour absorption percutanée comprenant : (a) une couche adhésive contenant un médicament qui contient de la mélatonine ou un sel pharmaceutiquement acceptable de celle-ci en tant que principe actif, un agent de solubilisation, un inhibiteur de cristallisation, un activateur d'absorption percutanée, un antioxydant, et un agent adhésif polymère ; (b) une couche de support ; et (c) une couche de libération,
dans laquelle la couche adhésive contenant un médicament contient, sur la base du poids total de la couche adhésive contenant un médicament, 3,0 à 20 % en poids de mélatonine ou du sel pharmaceutiquement acceptable de celle-ci, 1 à 30 % en poids de l'agent de solubilisation, 0,05 à 5 % en poids de l'inhibiteur de cristallisation, 1 à 30 % en poids de l'activateur d'absorption percutanée, 0,1 à 5 % en poids de l'antioxydant, et 50 à 94,85 % en poids de l'agent adhésif polymère,
dans laquelle l'agent de solubilisation est un ou deux ou plus choisis dans le groupe constitué de N-méthylpyrrolidone, dipropylène glycol, propylène glycol, carbonate de propylène, éthoxydiglycol, éther monoéthylique de diéthylène glycol, triacétine, citrate de triéthyle, triéthanolamine, trométhamine, bis-Tris, aminométhyl propanediol, aminoéthyl propanediol, et monooléate de polyoxyéthylène sorbitan,
dans laquelle l'inhibiteur de cristallisation pour inhiber la formation de cristaux du médicament est de la polyvinylpyrrolidone et/ou un copolymère de méthacrylate amino-acrylique,
dans laquelle l'activateur d'absorption percutanée est un dérivé aliphatique en C8-18,
dans laquelle le dérivé aliphatique en C8-18 est un ou deux ou plus choisis dans le groupe constitué d'alcool glycérol laurylique, alcool oléylique, myristate d'isopropyle, monooléate de sorbitan, monolaurate de propylène glycol, monooléate de propylène glycol, macrogolglycérides d'oléoyle, acide oléique, macrogol glycéride de lauroyle, macrogol glycéride de linoléoyle, dicaprylate/caprate de propylène glycol, monostéarate de sorbitan, monooléate de glycérol, monolaurate de glycérol, monolaurate de sorbitan, lactate de lauryle, triglycérides capryliques/capriques de PEG-8, monolaurate de polyoxyéthylène sorbitan, esters de PEG-8 d'huile de maïs, et esters de PEG-6 d'huile de maïs, et
dans laquelle l'antioxydant est un ou deux ou plus choisis dans le groupe constitué de butyl hydroxy toluène, butyl hydroxy anisole, galate de propyle, acide ascorbique, tocophérol, acétate de tocophérol, et palmitate d'ascorbyle.

2. La formulation pour absorption percutanée selon la revendication 1, dans laquelle l'agent adhésif polymère est un adhésif acrylique.

3. La formulation pour absorption percutanée selon la revendication 1, dans laquelle l'agent adhésif polymère est un agent adhésif polymère acrylique composé soit d'acrylate ou soit d'un copolymère d'acrylate et d'acétate de vinyle, dans laquelle l'agent adhésif polymère acrylique est un ou deux ou plus choisis dans le groupe constitué de (i) un n'ayant pas de groupe fonctionnel, (ii) un ayant un groupe hydroxyle (-OH) en tant que groupe fonctionnel, (iii) un ayant un groupe carboxyle (-COOH) en tant que groupe fonctionnel, et (iv) un ayant à la fois un groupe hydroxyle et un groupe carboxyle en tant que groupes fonctionnels.

4. La formulation pour absorption percutanée selon la revendication 1, dans laquelle l'agent adhésif polymère est un agent adhésif polymère acrylique composé soit d'acrylate soit d'un copolymère d'acrylate et d'acétate de vinyle, dans laquelle l'agent adhésif polymère acrylique est (i) un ayant un groupe carboxyle (-COOH) en tant que groupe fonctionnel, ou (ii) un ayant à la fois un groupe hydroxyle et un groupe carboxyle en tant que groupes fonctionnels.

5. La formulation pour absorption percutanée selon la revendication 1, dans laquelle l'agent adhésif polymère est un agent adhésif qui comprend un polymère hydrophobe, dans laquelle le polymère hydrophobe est un ou deux ou plus choisis dans le groupe constitué de polyisoprène, polyisobutylène, polybutadiène, copolymère de polystyrène-butadiène, copolymère de polystyrène-isoprène, copolymère séquencé de styrène-isoprène-styrène, copolymère séquence de styrène-butadiène-styrène, caoutchouc butyle, caoutchouc naturel, copolymère d'éthylène-acétate de vinyle, polysiloxane, et polymères à base d'acide méthacrylique.

6. La formulation pour absorption percutanée selon la revendication 5, dans laquelle l'agent adhésif polymère comprend en outre une résine donnant du collant et un plastifiant, et comprend 20 à 60 % en poids du polymère hydrophobe, 20 à 50 % en poids de la résine donnant du collant, et 2 à 30 % en poids du plastifiant.

7. La formulation pour absorption percutanée selon la revendication 1, dans laquelle la couche de support est une choisie dans le groupe constitué d'un film de téréphtalate de polyéthylène (PET), d'un film de polyéthylène (PE), d'un film de polypropylène (PP), d'un film d'éthylène-acétate de vinyle (EVA), d'un film de nylon, d'un stratifié de tissu non tissé/PET, d'un stratifié de PET/PE, et d'un stratifié de PET/EVA.

8. La formulation pour absorption percutanée selon la revendication 1, dans laquelle la couche de libération est une couche de libération obtenue par traitement de surface d'un choisi dans le groupe constitué d'un film de polyester, d'un film de chlorure de polyvinyle, d'un film de chlorure de polyvinylidène, d'un film de téréphtalate de polyéthylène (PET), d'un film de polyéthylène (PE), d'un stratifié de polyéthylène/papier, d'un stratifié de PET/PE, et d'un stratifié de PET/EVA, celui étant soumis à un traitement de surface étant traité avec un agent de traitement de silicone ou de fluor.
